# EUROPEAN PATENT SPECIFICATION

(11) **EP 2 239 003 B1**
(45) Date of publication and mention of the grant of the patent: **19.11.2014**
(21) Application number: 10158247.6
(22) Date of filing: 29.03.2010
(51) Int. Cl.: A61M 15/00

(54) **A counter for a dry powder inhaler device**
Zähler für eine Trockenpulverinhalatorvorrichtung
Compteur pour inhalateur de poudre sèche

(30) Priority: 05.05.2009 TR 200903493; 30.03.2009 TR 200902446
(43) Date of publication of application: 13.10.2010
(73) Proprietor: Arven Ilac Sanayi Ve Ticaret A.S., Istanbul 34460 (TR)
(72) Inventor: Toksöz, Ahmet, 34398, Istanbul (TR); Toksöz, Zafer, 34398, Istanbul (TR); Cifter, Ümit, 34398, Istanbul (TR)
(74) Representative: Sevinç, Erkan

(56) References cited:
- WO-A2-2004/009470
- DE-A1-102006 045 788
- US-A1- 2002 170 560
- US-A1- 2005 178 382

## Description

### Field of Invention

The present invention relates to inhaler devices allowing administration of dry powder inhalation medicaments, and more particularly to developments conducted on the counter component of such inhaler devices.

### Prior Art

Diseases such as asthma, bronchitis, and COLD (Chronic Obstructive Lung Disease) substantially decrease the quality of human life, despite the developments which have been carried out in the diagnosis and therapy thereof in the recent years. It has been proposed to administer medicaments via inhalers for optimizing the treatment of such diseases. The inhaler route of treatment is the most preferred one and it is expected to remain so, as a first option, in the future. The most important advantage of using medicaments via inhalation route is based on providing a more efficient therapy by making use of lesser medicaments, delivering higher concentration of medicaments to the respiration channels, and particularly decreasing the systemic side effects of medicaments. The most important causes of the lack of a satisfactory control of patients albeit the presence of quite efficient treatments against respiratory tract diseases are mentioned to be noncompliance to proper use of inhalers and insufficient obedience to doctor's recommended therapy.

Various inhalation devices for administering inhalation medicaments have been proposed until date. These devices are basically classified in two groups, i.e. metered dose inhalers and dry powder inhalers. These type of devices are structurally provided with basic components such as actuator, counter, housing, lid, lock, etc. On the other hand, powder inhalation medicaments are kept in reservoirs or containers such as blisters, capsules, etc. Blisters are structured from two basic parts, a main layer provided with cavities holding the medicament, and a strippable protective layer. It is very crucial with respect to user to meter or count each cavity consumed in devices containing blisters, and a number of counter embodiments of various types have already been proposed for this purpose.

One type of the current inhaler devices has the form of a disk, comprising multiple medicament compartments. These compartments comprise medicament cartridges, the medicaments are made ready for use after piercing by means of a needle, and administered by means of a mouthpiece. These devices further contain a disk-shaped counter, such that the number, i.e. numeric figure of consumed medicament cartridges is displayed by means of said counter. WO2008114034, WO2005004962 may be cited herein to exemplify such type of devices. Such devices can take a determined number of medicaments into the disk, so that an increase in the device volume as result of increasing the number of medicaments therein gives rise to a carrying/handling-, aesthetics- and use-related unpleasant outcomes. Additionally, they require the use of extra apparatuses for the counter.

WO 2004/009470 A2 discloses medicament dispenser for use with a medicament carrier having multiple distinct medicament doses carried thereby and an internal mechanism for dispensing the distinct medicament doses carried by said medicament carrier. The counting means is a distinct electronic counter unit that is reversibly receivable by the medicament dispenser.

DE 102006045788 discloses a dispenser for powder compounds, in particular medications from a blister pack, the rounded cavities of which can be brought by stepwise movement into an emptying position inside a dispenser housing and in said emptying position the medication can be opened by means of a needle and emptied by means of a vacuum stream which leads to a nozzle.

US 2002/170560 A1 discloses a dry powder inhaler for providing multiple doses of a pharmaceutical powder from blisters on a blister disk includes an actuator pivotably mounted on a base. Movement of the actuator from a first position to a second position drives the a dobber to open a blister. A tray retainer is moveable between opened and closed positions, and the actuator is moveable to a position at least partially overlying the tray retainer, when the tray retainer is in the closed position.

US 2005/178382 A1 discloses a dry powder inhaler comprising an inhaler housing having a mouthpiece and a slidablv extendable forward member that is movable between retracted and extended positions, held by the inhaler housing adjacent the mouthpiece. In the extended position, the forward member extends outward a distance beyond a forwardmost portion of the mouthpiece, and in the retracted position, a forwardmost portion of the forward member is positioned rearward of the forwardmost portion of the mouthpiece with an access portion of the mouthpiece accessible by a user.

The current inhaler devices are provided with various counter systems indicating the amount of consumed medicament powder. Patent applications WO2008110584, WO2005079727, US2003209239 disclose various counter embodiments. Said counter mechanisms comprise multiple components, with many of them having disks which function as a counter. Any malfunctioning of the components in counters can lead to an inaccurate metering of the amount or dose of medicament to be used, resulting in unresponsiveness to treatment, or even in more serious outcomes with respect to the patient. Other undesired outcomes also emerge, such as high costs, larger device volumes, operation-related problems, etc..

The complicated multicomponent structure of many of the existing counters cause application and use-related difficulties and prevent users from obtaining maximum possible efficiency.

In result, there is a need for development in the field of inhaler devices, providing high-accuracy operation, as well as advantages in terms of cost, volume, and usage.

### Objects and Brief Description of Invention

The present invention relates to a novel inhaler device for use with dry powder inhaling purposes, eliminating all aforesaid problems and brining additional advantages to the relevant prior art.

Accordingly, the main object of the present invention is to provide a counter embodiment, which is capable of operating accurately in a dry powder inhaler device and allowing easy and accurate display of the numbers on the blister strip out of the device.

Another object of the present invention is to provide a counter embodiment, which allows the blister strip in use to advance in an invariable distance pattern.

A further object of the present invention is to provide advantages in terms of cost and device volume, thanks to the present counter embodiment, which eliminates the requirement towards extra elements and components.

A dry powder inhaler device according to the present invention comprises an outer housing, an inner body disposed in the outer housing, a reservoir disposed at the first part of said inner body for receiving a blister strip with enumerated medicament-filled cavities, an actuating element moving the blister by rotating a gear set disposed in the inner body once said actuating element is slid or pushed into the interior of said outer housing, and a medicament release opening disposed at the second part of the outer housing on which a mouthpiece is provided. In addition, for providing an accurate view of, or for accurately displaying, the numbers on a blister from the inhaler device's exterior once said actuating element is pushed into the interior of the outer housing, the device according to the present invention further comprises an outer housing counter display gap disposed at the base part of said outer housing, an inner body counter display gap disposed at the lower part of said inner body so as to come to alignment with the outer housing counter display gap, an actuating element counter display gap disposed at the lower part of said actuating element so as to come to alignment with the inner body counter display gap, and an arc shaped surface piece to form a channel through which the blister is to be passed at the lower part of the inner body and to keep the blister at a desired stretched extent in said channel.

In a preferred embodiment according to the present invention, there is provided a positioning distance between the number of each cavity on the blister and the cavity of that respective number, this positioning distance being as large as the distance between the medicament release opening and the window formed by the counter display gaps.

In another preferred embodiment according to the present invention, one of the display gaps is provided with a lens magnifying the view of the blister.

### Brief Description of Figures

Figure 1 is an exploded schematic view of an illustrative embodiment according to the present invention.
Figure 2 is a perspective schematic view of the inner body according to the present invention.
Figure 3 is a cross-sectional schematic view of the subject inhaler device with a blister inserted.
Figure 4 is an illustrative view of the base part of the subject inhaler device.
Figure 5 is a cross-sectional schematic view of the counter display gaps and the reservoir of the subject inhaler device.

### Reference Numbers in Figures

- 1.: Outer housing
- 1.1: Outer housing counter display gap
- 2.: Inner body
- 2.1: Arc shaped surface piece
- 2.2: Inner body counter display gap
- 2.3: Reservoir
- 3.: Mouthpiece
- 3.1: Medicament release opening
- 4.: Blister
- 4.1: Cavity
- 5.: Actuating element
- 5.1: Actuating element counter display gap
- 5.2: Grip surface
- 6.: Inhaler
- 7.: Lid
- 8.: Gear set

### Detailed Description of the Invention

In the following detailed description, the inhaler device according to the present invention shall be described illustratively by making references to annexed figures, only to make it clear without imposing any restrictions thereon.

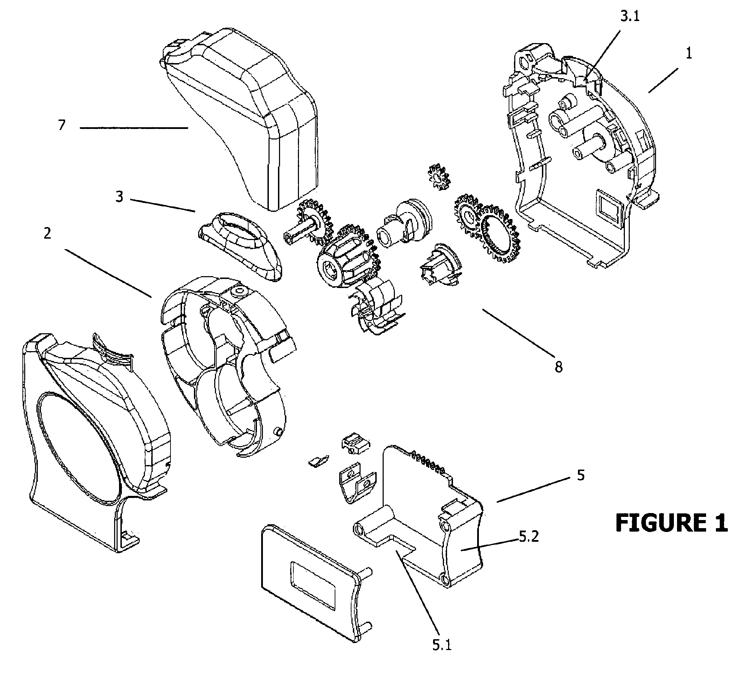

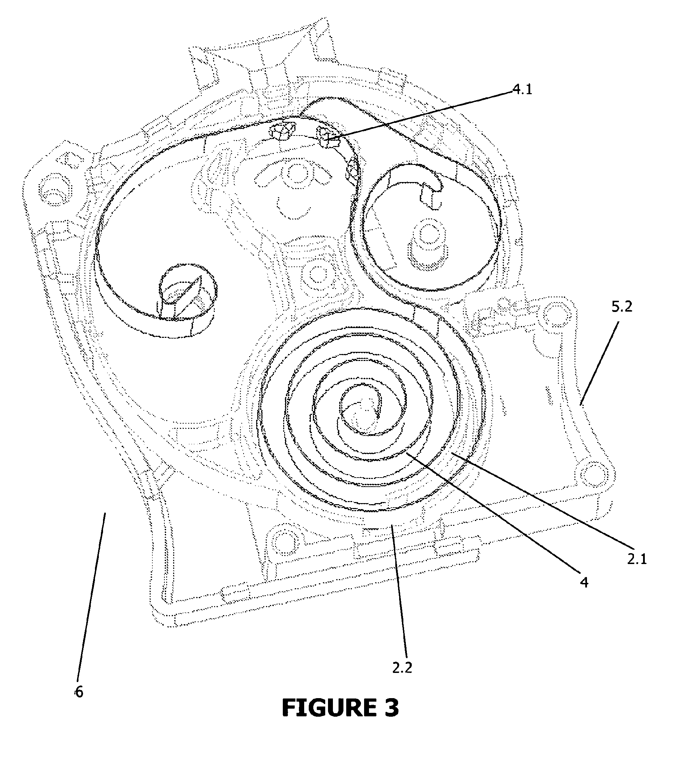

The outer housing (1) of the inhaler device according to the present invention, of which an exploded schematic view is given in figures 1 and 3, is made by assembling parts of the outer housing to each other, said housing having mutually-fitting structures and provided with fastening tabs at their periphery. The upper part of said outer housing (1) is provided with a medicament release opening (3.1) and a mouthpiece (3) disposed on said opening, so that the medicament included therein can be inhaled by a user. A lid (7) is provided, which preferably can be rotated as a result of being pivoted on the outer housing (1) and which can be closed so as to cover the mouthpiece (3) in order to keep clean said mouthpiece (3) when the inhaler device (6) is not in use and to prevent any unwanted substances from entering into the device (6). There is also formed an opening, referred to herein as the outer housing counter display gap (1.1), at the lower part of said outer housing (1).

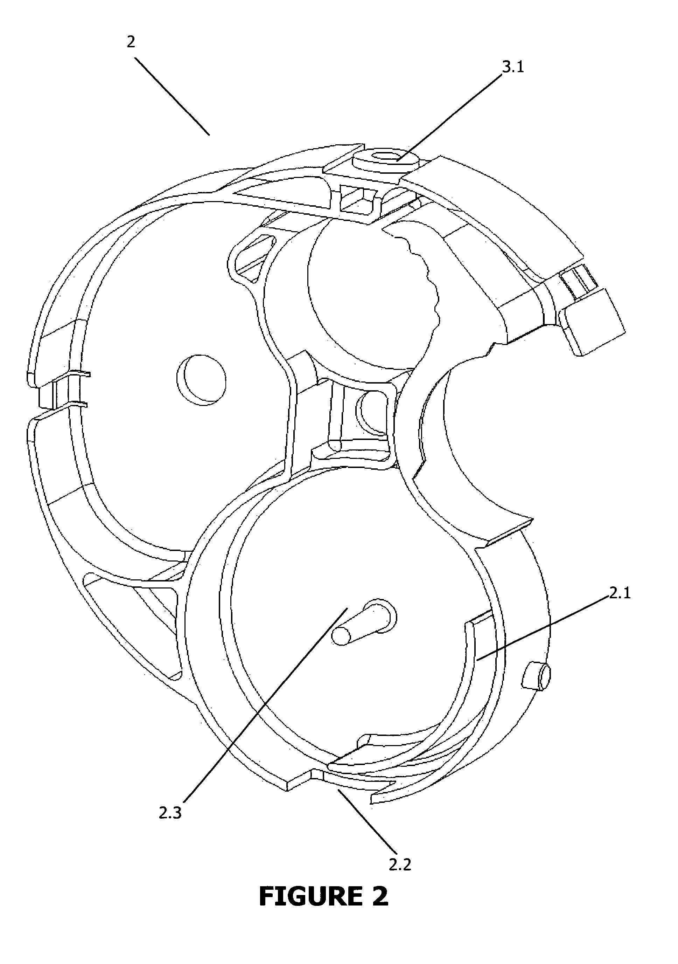

An inner body (2) is positioned in the interior of the outer housing (1), as illustrated in perspective in Figure 2. The lower part of said inner body (2) is embodied with a reservoir (2.3), into which an unused blister (4) is placed, said blister having medicament-filled cavities (4.1). An arc shaped surface piece (2.1) with a semicircular shape is formed at the lower part of the inner body (2) for producing a channel through which a blister (4) can be passed, whereas an opening, referred to herein as the inner body counter display gap (2.2), is produced on the inner body surface (2) under said piece (2.1). The other parts of said inner body (2) are in turn equipped with a gear set (8), which provide for the operation of the inhaler (6). Said gear set (8) also allows moving the blister (4) in one direction only.

An actuating element (5) is disposed at the lower lateral part of said outer housing (1), this actuating element being slid between the outer housing (1) and the inner body (2). A rectangular opening is formed at the lower side of the actuating element (5), referred to herein as the actuating element counter display gap (5.1). It is also feasible to dispose a lens at this site in order to optimize the view of the counter. It is possible to embody a counter mechanism which can reliably be used without requiring any additional elements.

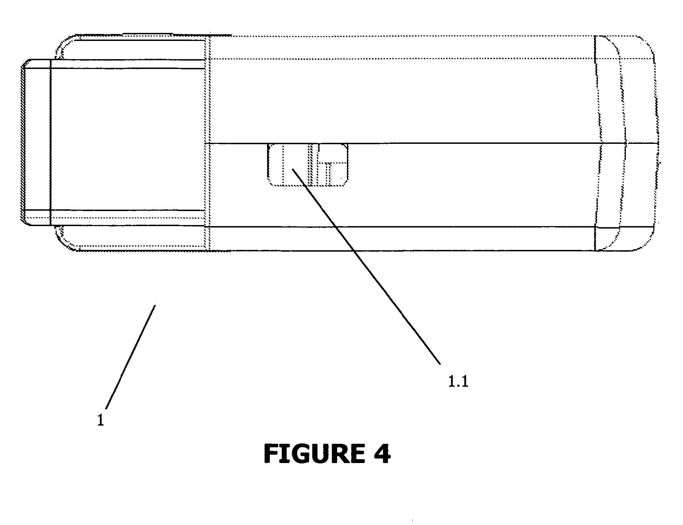

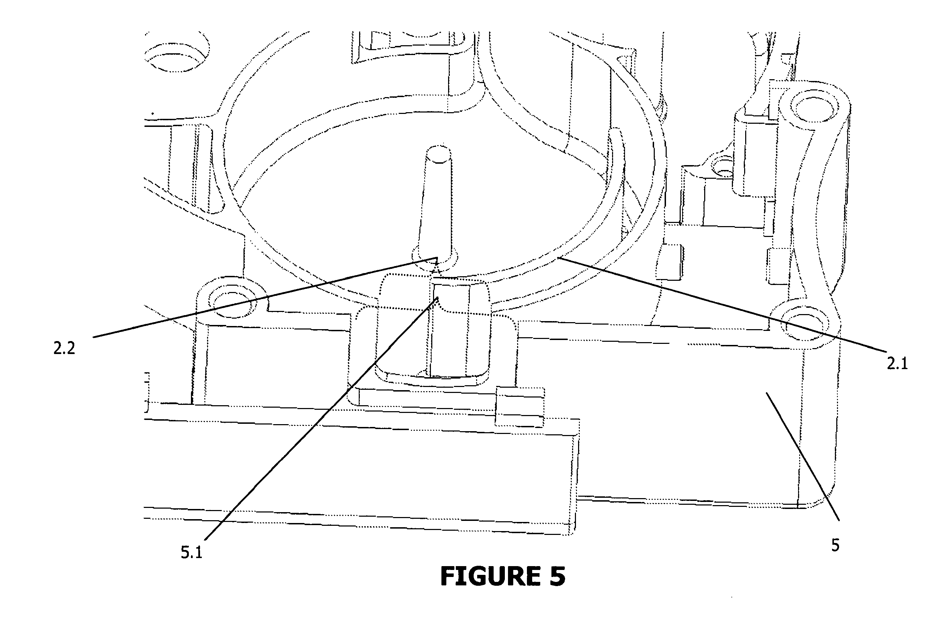

The openings referred to hereinabove, as the inner body counter display gap (2.2), as the actuating element counter display gap (5.1), and as the outer housing counter display gap (1.1), are superimposed so as to result in a window when the actuating element (5) is depressed, i.e. pushed in, as schematically illustrated in Figure 5. Thanks to this feature, when the device (6) illustrated in Figure 4 is viewed from its base, it becomes possible to see the cover layer, i.e. protective layer of the blister (4) present in the reservoir (2.3) of the inner body (2). The cover layer of the blister (4) is provided with numbers, indicating the sequential numbers of medicaments. There is provided, however, a positioning distance between the number of each cavity (4.1) and the cavity (4.1) of that respective number, as large as the distance between the medicament release opening (3.1) and the window formed by the counter display gaps (1.1, 2.2, 5.1). Thanks to this feature, when the actuating element (5) is depressed, and when the inhaler device (6) is viewed from its base or bottom, the number of the cavity (4.1) now arrived at the medicament release opening (3.1) can be seen. It is possible to make use of different signing or coloring methods in place of using the numeric figures on the blister's (4) protective layer, it being further feasible to apply these signs on the blister (4) by means of printing, labeling, using laser-based or even more advanced methods.

According to the structural details given above, the operation of the device (6) according to the present invention is as follows. Following the opening of lid (7), a force is exerted by the user to the grip surface (5.2) of the actuating element (5), thus to the actuating element (5) itself. Then the actuating element (5) is driven or slid or pushed into the interior of the housing (1). When this movement is transferred to the gear set (8) disposed in the inner body (2), the blister (4) is advanced through the channels of this body (2).

At the same time the relevant number on the blister (4) comes to alignment with the counter display gaps (1.1, 2.2, 5.1) of the outer housing, inner body, and actuating element, respectively. Thus, the user is able to see the number on the blister (4) properly further enabling him/her to see the amount of consumed medicaments by means of the counter mechanism embodied as described above. During the advancing movement, the blister (4) is kept at a desired stretched extent, so that the numbers come exactly to the same plane with the gaps (1.1, 2.2, 5.1), thanks to the arc shaped surface piece (2.1) formed at the inner body (2) surface. Thus, the numbers, i.e. numeric figures on the blister (4) are accurately read, and the probability of making mistakes is eliminated.

## Claims

1. A dry powder inhaler device (6), comprising an outer housing (1), an inner body (2) disposed in said outer housing (1), a reservoir (2.3) disposed at a first part of said inner body (2) for receiving a blister strip (4) with enumerated medicament-filled cavities (4.1), an actuating element (5) moving the blister (4) by rotating a gear set (8) disposed in the inner body (2) once said actuating element (5) is slid or pushed into the interior of said outer housing (1), and a medicament release opening (3.1) disposed at a second part of said outer housing (1) over which a mouthpiece (3) is provided; wherein the dry powder inhaler device (6) comprises;
an outer housing counter display gap (1.1) disposed at the base part of said outer housing (1),
an inner body counter display gap (2.2) disposed at said first part of said inner body (2) so as to come to alignment with said outer housing counter display gap (1.1), an arc shaped surface piece forming a channel through which the blister is to be passed at said first part of said inner body
**characterized in that** the inhaler device (6) further comprises;
an actuating element counter display gap (5.1) disposed at said first part of said actuating element (5) so as to come to alignment with said counter display gap (2.2),
the arc shaped surface piece (2.1) keeps the blister (4) at a desired stretched extent in said channel, whereby the numeric figures on said blister (4) can be accurately displayed to the exterior of said inhaler device (6) when said actuating element (5) is slid or pushed into the interior of said outer housing (1).

2. A dry powder inhaler device (6) according to Claim 1, **characterized in that** a positioning distance is provided between the numeric figure of each cavity (4.1) on the blister (4) and the cavity (4.1) of that respective numeric figure, this positioning distance being as large as the distance between the medicament release opening (3.1) and the window formed by the counter display gaps (1.1, 2.2, 5.1).

3. A dry powder inhaler device (6) according to any of the preceding claims, **characterized in that** one of the display gaps (1.1, 2.2) is provided with a lens which magnifies the appearance of the blister.

## Patentansprüche

1. Trockenpulverinhaliervorrichtung (6), umfassend
ein äußeres Gehäuse (1),
einen inneren Körper (2), der in dem äußeren Gehäuse (1) angeordnet ist,
ein Reservoir (2.3), das an einem ersten Teil des inneren Körpers (2) angeordnet ist zum Aufnehmen eines Blisterstreifens (4) mit spezifizierten, medikamentengefüllten Hohlräumen (4.1),
ein Antreibelement (5), welches den Blister (4) durch Drehen eines Getriebesatzes (8) bewegt, welches in dem inneren Körper (2) angeordnet ist, wenn das Antreibelement (5) in den Innenraum des äußeren Gehäuses (1) geschoben oder gedrückt wird, und eine Medikamentenausgabeöffnung (3.1), die an einem zweiten Teil des äußeren Gehäuses (1) angeordnet ist und über welcher ein Mundstück (3) angeordnet ist; wobei die Trockenpulverinhaliervorrichtung (6) umfasst:
eine Zähleranzeigelücke (1.1) des äußeren Gehäuses, welche an dem Basisteil des äußeren Gehäuses (1) angeordnet ist,
eine Zähleranzeigelücke (2.2) des inneren Körpers, die an dem ersten Teil des inneren Körpers (2) so angeordnet ist, dass sie in Ausrichtung mit der Zähleranzeigelücke (1.1) des äußeren Gehäuses kommt,
ein bogenförmiges Oberflächenstück, das einen Kanal bildet, durch welchen der Blister an dem ersten Teil des inneren Körpers durchzuführen ist,
**dadurch gekennzeichnet,**
**dass** die Inhaliervorrichtung (6) außerdem aufweist:
eine Zähleranzeigelücke (5.1) des Antreibelements, welche an dem ersten Teil des Antreibelements (5) so angeordnet ist, dass sie in Ausrichtung mit der Zähleranzeigelücke (2.2) kommt,
**dass** das bogenförmige Oberflächenstück (2.1) den Blister (4) an einem gewünschten getreckten Ausmaß in dem Kanal hält, wodurch die numerischen Angaben auf dem Blister (4) präzise zu der Umgebung der Inhaliervorrichtung (6) angezeigt werden können, wenn das Antreibelement (5) in den Innenraum des äußeren Gehäuses (1) geschoben oder gedrückt ist.

2. Trockenpulverinhaliervorrichtung (6) nach Anspruch 1,
**dadurch gekennzeichnet,**
**dass** eine Positionierentfernung zwischen der numerischen Angabe von jedem Hohlraum (4.1) an dem Blister (4) und dem Hohlraum (4.1) der jeweiligen numerischen Angabe vorhanden ist, wobei diese Positionierentfernung so groß ist wie die Entfernung zwischen der Medikamentenausgabeöffnung (3.1) und dem Fenster, das durch die Zähleranzeigelücken (1.1, 2.2, 5.1) gebildet ist.

3. Trockenpulverinhaliervorrichtung (6) nach einem der vorhergehenden Ansprüche,
**dadurch gekennzeichnet,**
**dass** eine der Anzeigelücken (1.1, 2.2) mit einer Linse versehen ist, welche die Erscheinung des Blisters vergrößert.

## Revendications

1. Dispositif inhalateur de poudre sèche (6) comprenant un boîtier externe (1), un corps interne (2) disposé dans ledit boîtier externe 1, un réservoir (2, 3) disposé dans une première partie dudit corps interne (2), réservoir, qui est destiné à recevoir une plaquette de blisters (4) avec des cavités (4.1) énumérées, remplies de médicament, un élément d'actionnement (5) déplaçant le blister (4) en mettant en rotation un jeu de pignons (8) disposé dans le corps interne (2) une fois que l'élément d'actionnement (5) est coulissé ou poussé à l'intérieur dudit boîtier externe (1) et une ouverture de libération de médicament (3.1) disposée dans une seconde partie dudit boîtier externe (1), ouverture, au-dessus de laquelle est prévu un embout buccal, le dispositif inhalateur de poudre sèche (6) comprenant :
un espace d'affichage compteur du boîtier externe (1.1) disposé au niveau de la partie de base dudit boîtier externe (1),
un espace d'affichage compteur du corps interne (2.2) disposé au niveau de ladite première partie dudit corps interne (2) de sorte à venir en alignement avec ledit espace d'affichage compteur du boîtier externe (1.1),
une pièce de surface en forme d'arc formant un canal à travers lequel le blister est à passer dans ladite première partie dudit corps intérieur,
**caractérisé en ce que** le dispositif inhalateur (6) comprend en outre :
un espace d'affichage compteur de l'élément d'actionnement (5.1) disposé au niveau de ladite première partie dudit élément d'actionnement (5) de sorte à venir s'aligner avec ledit espace d'affichage compteur (2.2),
et **en ce que** la pièce de surface en forme d'arc (2.1) maintient le blister (4) sur une étendue d'étirement souhaitée dans ledit canal, ce qui permet d'afficher les chiffres numériques sur ledit blister (4) avec précision à l'extérieur dudit dispositif inhalateur (6) lorsque ledit élément d'actionnement (5) est coulissé ou poussé à l'intérieur dudit boîtier externe (1).

2. Dispositif inhalateur de poudre sèche (6) suivant la revendication 1, **caractérisé en ce qu'**une distance de positionnement est prévue entre le chiffre numérique de chaque cavité (4.1) sur le blister (4) et la cavité (4.1) du chiffre numérique respectif, cette distance de positionnement étant aussi grande que la distance entre l'ouverture de libération du médicament (3.1) et la fenêtre formée par l'espace d'affichage compteur (1.1, 2.2, 5.1).

3. Dispositif inhalateur de poudre sèche (6) suivant une quelconque des revendications précédentes, **caractérisé en ce que** l'un des espaces d'affichage (1.1, 2.2) est muni d'une loupe qui grossit la vision du blister.
